# EUROPEAN PATENT APPLICATION

(11) **EP 2 689 706 A2**
(43) Date of publication of application: **29.01.2014**
(21) Application number: 12190862.8
(22) Date of filing: 31.10.2012
(51) Int. Cl.: A61B 1/00, A61M 25/00, A61B 1/12, A61B 19/02

(54) **A storage receptacle for medical instruments**

(30) Priority: 31.10.2011 GB 201118735
(71) Applicant: Partners for Endoscopy Limited, Stoke on Trent ST4 3PE (GB)
(72) Inventor: Hartley, Robert John, Fenton, Aberdeenshire ST4 3PB (GB)
(74) Representative: Jones, David Alan

(57) **Abstract**

A storage receptacle (1) for storage of a medical instrument such as an endoscope comprises a base (4) and a plurality of upstanding walls (6) extending from the base (4) defining an enclosure for receiving and storing a medical instrument. A cover member (20) is provided for closing the enclosure. The cover member (20) comprises a first indicia provided on a first face indicative of an unused condition and a second indicia provided on an opposing face of the cover (20) indicative of a used condition and the cover member (20) is configured to attach to and close the enclosure in a first orientation in which the first face is outwardly facing and in a second orientation in which the second face is outwardly facing. The storage receptacle (1) therefore permits the sterile condition of the contents to be readily discerned by the user and prevents dirty instruments from being returned to storage in an unsterile condition.

## Description

The present invention relates to storage receptacle for storage of a medical instrument, and in particular a disposable moulded pulp receptacle.

Medical instruments such as endoscopes, which are required to be sterilised prior to use in surgery, are typically sterilised and subsequently stored in a sterile storage environment until required. It is common practice for the instruments to be stored in a receptacle such as a tray, which must also be sterile. Such trays are typically formed of a plastic material, although metal trays are also known. When required the instrument is carried to the point of use in the tray and following use the dirty instrument is returned to the tray. Both the tray and instrument must then be sterilised before being placed back in sterile storage.

It is important in order to ensure that the instrument presented for use has been sterilised that following use the dirty instrument is identified as such and not returned to storage risking the later use of an unsterile instrument.

Furthermore, as health authorities strive to cut expenditure, there is a need to avoid the requirement for sterilisation of both the medical instrument and the storage receptacle in which the instrument is stored, with the sterilisation of the storage receptacle undesirably requiring additional time and expense.

It is therefore desirable to provide an improved storage receptacle which addresses the above described problems and/or which offers improvements generally.

According to the present invention there is provided a storage receptacle as described in the accompanying claims. There is also provided a method of storage and sterilisation of medical instruments as described in the accompanying claims.

In an embodiment of the invention there is provided a storage receptacle for storage of a medical instrument, the storage receptacle comprising a base; a plurality of upstanding walls extending from the base defining a enclosure for receiving and storing a medical instrument; and a cover member for closing said enclosure. The cover member comprises a first indicia provided on a first face indicative of an unused condition and a second indicia provided on an opposing face of the cover indicative of a used condition and the cover member is configured to attach to and close the enclosure in a first orientation in which the first face is outwardly facing and in a second orientation in which the second face is outwardly facing. The storage receptacle therefore permits the sterile condition of the contents to be readily discerned by the user and prevents dirty instruments from being returned to storage in an unsterile condition. The use of the reversible lid provides a convenient means of indication which obviates the requirement for an additional dedicated means of indication, with the lid providing the dual function of closing the receptacle and presenting the indicia.

The storage receptacle may comprise at least one connection member configured to connect the cover member to the enclosure in both the first and second orientations. While it possible that the lid may be supported in an unconnected manner on the receptacle is preferred that the lid is connected thereto to ensure it is retained on the receptacle in transit and to prevent the lid from being accidentally flipped to present the incorrect indicia.

The at least one connection member may comprise a tab extending from the cover member, with the enclosure including at least one elongate slot corresponding to and configured to receive the at least one tab, the tab being movable between a first connection position for connecting the cover member in the first orientation and a second position for connecting the cover member in the second orientation. The tabs provide an effect connection means which obviates the requirement for adhesives and enables repeated disconnection and reconnection.

The receptacle is preferably formed from a moulded pulp material and preferably from a maceratable, dried moulded pulp paper, which is advantageously inexpensive and disposable. In this way the receptacle may be disposed of following use thereby obviating the requirement for additional sterilisation.

The upstanding walls may comprise upper edges defining a rim and at least a portion of the rim is configured to support the cover member in both first and second orientations, the cover member cooperating with the rim to close the enclosure. The closure member is configured to overlap the inner periphery defining the opening and be supported on the rim thereby ensuring

The rim preferably includes a recess extending at least partially around its inner peripheral edge configured to receive a corresponding portion of the lid.

The enclosure is preferably configured to receive an endoscope.

The storage receptacle preferably further comprises a liquid receptacle located within the enclosure and integrally moulded therewith. The liquid receptacle advantageously enables the storage receptacle to act as a receptacle for cleansing solution to initially cleanse the endoscope immediately following use.

The liquid receptacle preferably comprises a wall extending upwardly from the base defining a liquid receiving volume, the wall being located inwardly of the plurality of walls defining the enclosure. The liquid receptacle is therefore contained within the enclosure and covered by the closure member during storage.

At least the liquid receptacle portion of the storage receptacle may be formed from a liquid resistant moulded pulp material, although preferably the entire storage receptacle is liquid resistant.

The wall may define a substantially truncated conical shape tapering inwardly away from the base. The liquid receptacle may include an inner wall which meets the outer wall at its upper edge and tapers inwardly in a downwards direction away from the upper edge. This enables the inner wall to sit within the liquid receptacle of a further storage receptacle when stacked in a nested arrangement.

The liquid receptacle preferably comprises an upper edge arranged to support the lid when it is attached to the enclosure, and which preferably extends from the base to a height substantially equal to the height of the portion of the upstanding walls defining the enclosure supporting the lid.

In a further embodiment of the invention there is provided a method of storage and sterilisation of medical instruments comprising placing a sterilised medical instrument in a storage receptacle having a plurality of upstanding walls defining an enclosure; closing the enclosure with a cover member such that a first face of the closure member displaying an indicia indicative of an unused condition is outwardly facing; returning the medical instrument to the storage receptacle following use and closing storage receptacle with the cover member such that a second face of the cover member displaying an indicia indicative of a used condition is outwardly facing.

In another embodiment there is provided a storage receptacle for storage of a medical instrument, the storage receptacle comprising a base; a plurality of upstanding walls extending from the base defining an enclosure for receiving and storing a medical instrument; and a liquid receptacle located within the enclosure. The enclosure may comprise a lid for closing the enclosure.

The liquid receptacle preferably comprises a wall extending upwardly from the base defining a liquid receiving volume, the wall being located inwardly of the plurality of walls defining the enclosure.

The storage receptacle may formed from moulded pulp and preferably macerated moulded pulp, and the liquid receptacle is integrally moulded therewith.

Preferably at least the liquid receptacle portion of the storage receptacle is formed from a liquid resistant moulded pulp material.

The wall of the liquid preferably defines a substantially truncated conical shape tapering inwardly away from the base. This provides optimum structural rigidity, and optimises space for the endoscope within the tray due it tapering towards the top of the enclosure.

Preferably the liquid receptacle comprises an upper edge arranged to support a lid when it is attached to the enclosure.

The present invention will now be described by way of example only with reference to the following illustrative figures in which:
Figure 1 shows a storage receptacle according to an embodiment of the present invention with the lid removed;
Figure 2 shows shows a storage receptacle according to a further embodiment of the present invention;
Figure 3 shows a storage receptacle according to an embodiment of the present invention with the lid secured in an orientation indicating a sterilised condition;
Figure 4 shows the first 'clean' face of a lid according to an embodiment of the invention; and
Figure 5 shows a second 'dirty' face of the lid of Figure 4.

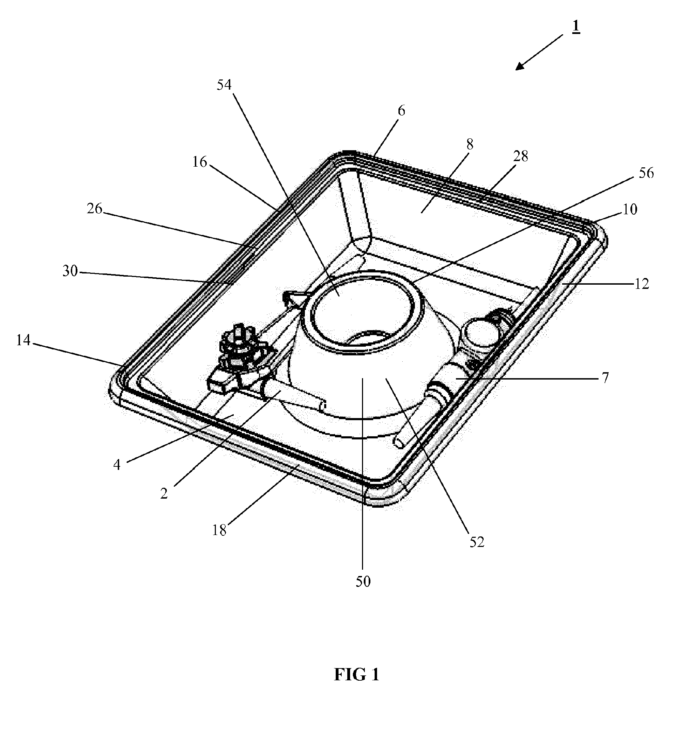

Referring to Figure 1, a storage container 1 is provided for the storage of medical instruments such as endoscopes 2. The storage receptacle 1 comprises a base 4 and four upstanding walls 6 extending upwardly from the base defining an enclosure 8. The walls 6 include upper edges 10 which form a perimeter defining an opening 10 to the enclosure 8.

The upstanding walls 6 taper outwardly away from the base 4 such that the opening 12 has a larger area than the base 4. This tapered configuration enables the storage container 1 to be nestabley stacked within another corresponding storage container.

The enclosure 8 is substantially rectangular in shape when viewed from above having longer side edges 16 and shorter end edges 18, although it will be appreciated that any suitable configuration comprising a base and a wall defining an enclosure may be utilised. The walls 6 extend in a substantially horizontal at their upper edge 10 direction to define a flanged section 14 forming a lip or a rim. The flange section 14 reinforces and provides strength to the upper edges of the walls 6. The flange section 14 includes a recess 26 defined around its inner edge forming a stepped ledge at a lower level to the uppermost surface of the flange 14. The recess 26 extends around the entire inner periphery of the flange section 14.

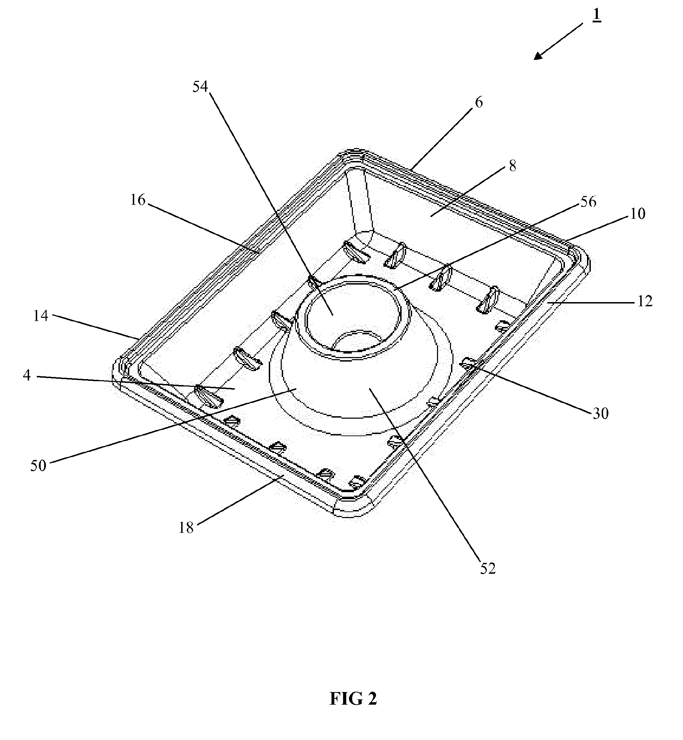

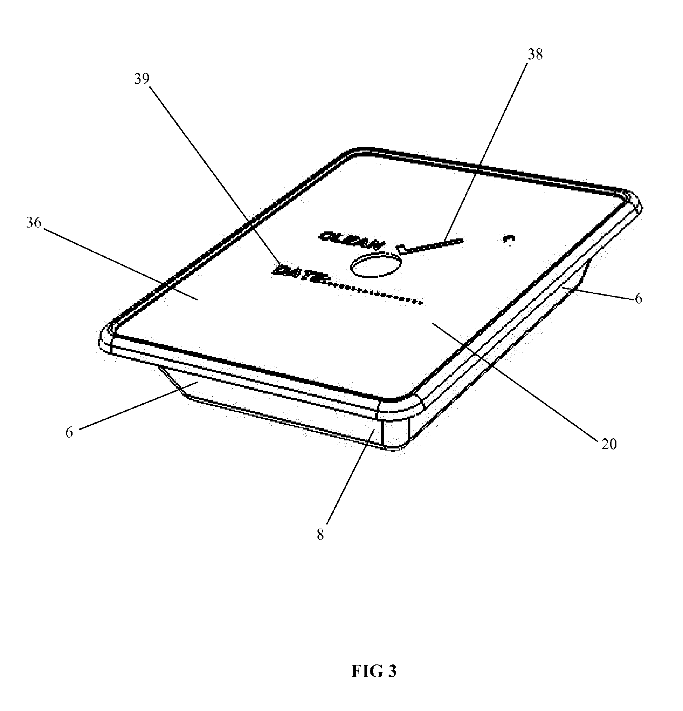

A lid 20 is provided as shown in Figure 2 for covering and closing the opening 12 of the enclosure 8, as shown in Figure 3. The lid 20 is a rigid planar member preferably formed from cardboard or a similar disposable material. The lid 20 is sized to overlap the opening 12 defined by the inner perimeter of the flange section 14 and to be supported on the surface of flange section 14 within the recess 26 on the upwardly facing surface thereof. The lid 20 is rectangular sized and shaped to correspond substantially to the size and shape of the outer peripheral edge of the recess 26 such that it fits closely within the recess. The depth of the recess 26 is greater than the thickness of the lid 20 such that the step defined by the recess 26 laterally retains the lid 20. While the recess 26 I shown to extend around the entire inner periphery of the flange section 14, in an alternative embodiment a plurality of recesses may be provided at discrete points arounf the inner periphery, with the lid including corresponding projection configured to be received in the discrete recess sections.

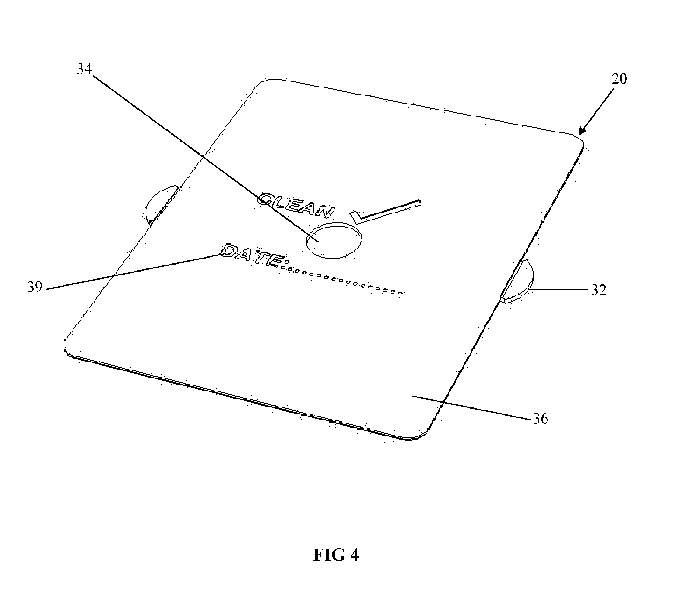

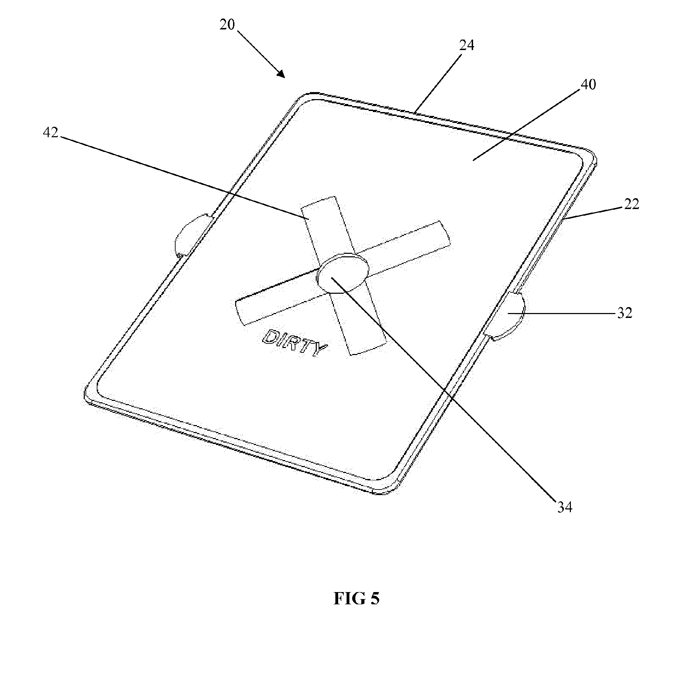

A pair of slots 30 are formed within the upwardly facing surface of the recess 26 and are located centrally along the portion of the recess 26 extending along the side edge of the enclosure 8. The slots 30 are sized and positioned to receive corresponding tabs 32 extending from the side edges 27 of the lid 20, as shown in Figures 4 and 5. The tabs 32 are bendable about a living hinge and are bendable to an orientation substantially perpendicular to the planer surface defined by the main body of the lid 20 such that when the lid 20 is oriented horizontally the tabs 32 extend downwardly. As such, when the lid 20 is oriented for placement on the storage container 1 the tabs are bent to extend substantially vertically downwards and are received in and extend downwardly through the slots 30. When the tabs 32 are received in the slots 30 the slots 30 and tabs 32 cooperate to hold and retain the lid 20 into position on top of the storage container 1. A central aperture e34 is defined in the lid 20 into which a user may place a finger to enable them to easily lift and remove the lid 20. Alternatively, a scalloped section may be provided at one or more of the corners of the lid 20 which define apertures into which a user may place their fingers to lift the lift 20.

As shown in Figures 3 and 4, a first face 36 of the lid 20 includes an indicia 38 indicating that the contents of the tray have been sterilised. In the arrangement shown the indicia 38 comprises the word "clean" in combination with a tick symbol (√) to indicate that the contents of the tray have been cleaned and sterilised. In addition, a further printed section 39 is provided to prompt the user to mark the lid 20 with the date on which the contents were sterilised and placed within the container 1.

Following sterilisation an endoscope 2 is placed into the enclosure 8 and the lid 20 is oriented such that the first face 36 and the corresponding first indicia 38 are facing upwardly. The tabs 32 of the lid 20 are bent such that they extend perpendicular to the planer body of the lid 20 in a downward direction away from the first face 36 when the first face 36 is facing upwardly. The tabs 32 are inserted into the corresponding slots 30 to retain the lid 20 in position. With the lid so secured to the enclosure 8 the first face 36 is outwardly facing from the container such that it is outwardly facing and externally visible. In this configuration the indicia 38 of the first face 36 is also outwardly facing and clearly visible providing an indication to the user of the sterilised and clean status of the contents of the storage container.

The opposing second face 40 of the container 1 is shown in Figure 5 and comprises a second indicia 42 indicative of the contents of the storage container 1 being in a 'used' condition being dirty and unsterilized. In the arrangement shown in Figure 5 the indicia 42 comprises a cross (X) and the word "dirty". When the endoscope or other instrument 2 has been used it is placed back in the storage container 1 in a dirty condition. The lid 20 is then oriented such that the second face 40 and the corresponding second indicia 42 are facing upwardly. The tabs 32 of the lid 20 are bent such that they extend perpendicular to the planer body of the lid 20 in a downward direction away from the second face 40 when the second face 40 is facing upwardly. The tabs 32 are inserted into the corresponding slots 30 to retain the lid 20 in position. With the lid so secured to the enclosure 8 the second face 40 is outwardly facing from the container such that it is outwardly facing and externally visible. In this configuration the second indicia 42 of the second face 40 is outwardly facing and clearly visible providing an indication to the user of the sterilised and clean status of the contents of the storage container.

The first indicia 38 and the second indicia 42 shown in figures 2 and 3 are shown by way of example. It would be appreciative of any suitable indicia may be provided to indicate the corresponding clean and dirty conditions of the storage container 1. For example, the first face 36 may be partially or completely covered in a first colour, for example green indicating the clean condition while the opposing second face 40 may be partially or completed covered red to indicate the dirty condition of the storage container 1. Similarly any other suitable indicia may be provided with the only requirement that first indicia 38 and second indicia 42 are capable of being easily distinguished from each other and associated with clean and dirty conditions respectively.

The storage container 1 of the present invention is formed from a moulded pulp material formed from a masseratable dried moulded pulp paper. The container 1 is formed as a single piece unitary moulded item. The use of a moulded pulp paper material enables the storage container to be manufactured at a very low cost as compared to plastic or materials. Moulded pulp products are also easily and readily recyclable. As such, the combination of the low cost and recyclability of the storage container 1 means the storage container 1 may be used as a single use disposable item. The storage container 1 may be provided to a user which would typically be a health trust or other medical organisation, in a pre sterilised condition enabling the endoscope 2 or other instrument to be placed directly into the storage container 1 without the requirement for the user to sterilise the container 1. Following use the container 1 may be disposed of as the endoscope 2 sterilised and placed into a fresh replacement container 1 stored within the sterile storage environment. As such, the requirement for the user to sterilise the storage container following use is entirely obviated. In addition, the use of the first and second indicia ensure that storage container 1 having dirty unsterilized instruments within are not returned to storage for use and instead are easily and readily identified as requiring sterilisation of the instrument and disposal of the storage container and lid 20.

Immediately following use of an endoscope 2, and prior to a thorough sterilization procedure, health guidelines recommend that an initial clean down or 'suck through' of the endoscope 2 to remove at an early stage soils, proteins and body fluids. Such early removal is critical as to allow these to dry can result in the blockage of air/water channels and allow a biofilm to proliferate in the suction/biopsy channel that is impossible to remove. For this initial clean down a enzymatic cleaning solution is typically used. This solution is provided in sealed pouches which are emptied into a receptacle at the point of use. This receptacle must then also be sterilised in addition to the endoscope and its storage tray. To obviate the requirement for a further container and a yet further and unnecessary sterilising procedure the container 1 of the present invention includes a liquid receptacle 50 for containing sterilizing solution. The liquid receptacle 50 is substantially formed as a truncated cone which tapers inwardly away from the base 4 in an upward direction. The receptacle 50 includes an inner wall 54 and an outer wall 52 which intersect at the upper edges to define the upper rim 56 of the liquid receptacle 50. The outer wall 52 defines the truncated conical shape of the liquid receptacle 50 having the wider base which tapers inwardly away from the base 40.

The inner wall 54 tapers inwardly in a downward direction away from the upper rim 56 and defines an inverted truncated cone. Alternatively the inner wall 54 may be substantially hemispherical and bowl-like in configuration. At least the inner wall 54 comprises a liquid resistant material such that the storage receptacle 50 may hold and retain liquid without it permeating through the container 1. Preferably the entire container 1 is formed from a water resistant moulded pulp product. Alternatively, the inner wall 54 may be separately treated or formed to provide liquid resistance. A cleaning solution is provided with the storage container 1 within a sealed bag which is preferably stored within the liquid receptacle 50. At the point of use the cleaning solution is released from the bag and poured into the liquid receptacle 50 which holds the cleaning solution for use in initial cleaning of the endoscope 2 following use.

The height of the upper rim 56, defined by the height of the inner wall 54 and outer wall 52 is selected such that it is substantially equal to the height of the upwardly facing surface of the recess 26 supporting the lid 20. The height of the upper rim 56 therefore corresponds to the height of the lower surface of the lid 20 when it is placed on a storage container 1 and received in the recess 26. The upper rim 56 thereby provide a central support for the lid 20 to prevent sagging of the lid 20. This enables the lid 20 to be more firmly held and secured on the storage container 1.

Whilst endeavouring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

It will be appreciated that in further embodiments various modifications to the specific arrangements described above and shown in the drawings may be made. For example the enclosure may be of any shape and configuration suitable to house and contain a medical instrument and to be closed by an appropriate lid. Furthermore, while the invention is described in the main for use with an endoscope it may equally be applied for the storage of an item which must be cleaned and/or sterilised following use. In addition, while the truncated conical liquid receptacle is described in combination with the reversible lid, it will be appreciated that in other embodiments these features may be provided independently of each other and need not be provided in combination. Furthermore, while preferable it is conceived that the liquid may be provided in configurations other than a truncated cone.

## Claims

**1.** A storage receptacle for storage of a medical instrument, the storage receptacle comprising:
a base;
a plurality of upstanding walls extending from the base defining a enclosure for receiving and storing a medical instrument; and
a cover member for closing said enclosure;
wherein the cover member comprises a first indicia provided on a first face indicative of an unused condition and a second indicia provided on an opposing face of the cover indicative of a used condition and the cover member is configured to attach to and close the enclosure in a first orientation in which the first face is outwardly facing and in a second orientation in which the second face is outwardly facing.

**2.** A storage receptacle according to claim 1 comprising at least one connection member configured to connect the cover member to the enclosure in both the first and second orientations.

**3.** A storage receptacle according to claim 2 wherein the at least one connection member comprises a tab extending from the cover member and the enclosure includes at least one slot corresponding to and configured to receive the at least one tab, the tab being movable between a first connection position for connecting the cover member in the first orientation and a second position for connecting the cover member in the second orientation.

**3.** A storage receptacle according to any preceding claim wherein the upstanding walls comprise upper edges defining a rim and at least a portion of the rim is configured to support the cover member in both first and second orientations, the cover member cooperating with the rim to close the enclosure.

**4.** A storage receptacle according to any preceding claim further comprising a liquid receptacle located within the enclosure and integrally moulded therewith.

**5.** A storage receptacle according to claim 4 wherein the liquid receptacle comprises a wall extending upwardly from the base defining a liquid receiving volume, the wall being located inwardly of the plurality of walls defining the enclosure.

**6.** A storage receptacle according to claim 5 wherein at least the liquid receptacle portion of the storage receptacle is formed from a liquid resistant moulded pulp material.

**7.** A storage receptacle according to claim 5 or 6 wherein the wall defines a substantially truncated conical shape tapering inwardly away from the base.

**8.** A storage receptacle according to claim 7 wherein the liquid receptacle extends from the base to a height substantially equal to the height of the portion of the upstanding walls defining the enclosure supporting the lid.

**9.** A method of storage and sterilisation of medical instruments comprising:
placing a sterilised medical instrument in a storage receptacle having a plurality of upstanding walls defining an enclosure;
closing the enclosure with a cover member such that a first face of the closure member displaying an indicia indicative of an unused condition is outwardly facing;
returning the medical instrument to the storage receptacle following use and closing storage receptacle with the cover member such that a second face of the cover member displaying an indicia indicative of a used condition is outwardly facing.

**10.** A storage receptacle for storage of a medical instrument, the storage receptacle comprising:
a base;
a plurality of upstanding walls extending from the base defining an enclosure for receiving and storing a medical instrument; and
a liquid receptacle located within the enclosure.

**11.** A storage receptacle according to claim 10 wherein the liquid receptacle comprises a wall extending upwardly from the base defining a liquid receiving volume, the wall being located inwardly of the plurality of walls defining the enclosure.

**12.** A storage receptacle according to claim 10 or 12 wherein the storage receptacle is formed from moulded pulp and the liquid receptacle is integrally moulded therewith.

**13.** A storage receptacle according to claim 12 wherein at least the liquid receptacle portion of the storage receptacle is formed from a liquid resistant moulded pulp material.

**14.** A storage receptacle according to any one of claim 11 to 13 wherein the wall of the liquid defines a substantially truncated conical shape tapering inwardly away from the base.

**15.** A storage receptacle according to any one of claims 10 to 14 wherein the liquid receptacle comprises an upper edge arranged to support a lid when it is attached to the enclosure.
